# EUROPEAN PATENT APPLICATION

(11) **EP 4 026 581 A1**
(43) Date of publication of application: **13.07.2022**
(21) Application number: 20870787.7
(22) Date of filing: 07.09.2020
(51) Int. Cl.: A61M 21/02, F24F 11/66, A61B 5/00, A61B 5/107, A61F 5/56

(54) **ENVIRONMENT CONTROL DEVICE**

(30) Priority: 30.09.2019 JP 2019179913
(71) Applicant: Daikin Industries, Ltd., Osaka-shi, Osaka 530-8323 (JP); OSAKA UNIVERSITY, Suita-shi Osaka 565-0871 (JP)
(72) Inventor: TSUBOI Chiaki, Osaka-shi, Osaka 530-8323 (JP); HASHIMOTO Satoshi, Osaka-shi, Osaka 530-8323 (JP); HORI Shouta, Osaka-shi, Osaka 530-8323 (JP); KATO Takafumi, Suita-shi, Osaka 565-0871 (JP)
(74) Representative: Hoffmann Eitle
(86) International application number: PCT/JP2020/033776
(87) International publication number: WO 2021/065360

(57) **Abstract**

An environment control device (1) includes a determination unit (10) and a supply unit (20). The determination unit (10) determines the type of symptom that disturbs sleep of a sleeper. The supply unit (20) supplies the sleeper with a stimulus that reduces the symptom, according to the type of the symptom, disturbing the sleep, determined by the determination unit (10).

## Description

### TECHNICAL FIELD

The present disclosure relates to an environment control device.

### BACKGROUND ART

Symptoms that disturb sleep of a sleeper, such as snoring and bruxism, may, for example, cause frequent awakenings of the sleeper during sleep and thus may deteriorate their sleep quality if such symptoms occur during sleep. Some methods have been proposed to prevent or reduce snoring and bruxism.

As a known technique, Patent Document 1, for example, proposes a method using a snore detector that detects a snore. When a snore is detected, random airflow, 1/f airflow, aromatic airflow, or the like is supplied to a sleeper to stop the snore.

### CITATION LIST

### PATENT DOCUMENT

Patent Document 1: Japanese Unexamined Patent Publication No. H4-146746

### SUMMARY OF THE INVENTION

### TECHNICAL PROBLEM

However, the known technique does not take the cause of the snore into account in preventing the snore. Thus, the effect on reduction or stop of the snore, and hence the effect on improvement of sleep quality, are less reliable.

It is an object of the present disclosure to reduce symptoms that disturb sleep of a sleeper and thereby improve their sleep quality.

### SOLUTION TO THE PROBLEM

A first aspect of the present disclosure is directed to an environment control device including: a determination unit (10) configured to determine a type of a symptom that disturbs sleep of a sleeper; and a supply unit (20) configured to supply the sleeper with a stimulus that reduces the symptom, according to the type of the symptom determined by the determination unit (10).

According to the first aspect, the determination unit (10) determines a type of a symptom that disturbs sleep of a sleeper, and the supply unit (20) supplies the sleeper with a stimulus suitable for the type of the symptom. This configuration can reduce a symptom that disturbs sleep in consideration of the cause of the symptom. The sleep quality can thus be improved.

A second aspect of the present disclosure is an embodiment of the first aspect. In the second aspect, the symptom includes at least snoring, and the determination unit (10) determines whether the snoring is due to nasal congestion or due to constriction of the throat.

According to the second aspect, it is possible to select stimuli suitable for the snoring due to nasal congestion and the snoring due to constriction of the throat.

A third aspect of the present disclosure is an embodiment of the second aspect. In the third aspect, when the determination unit (10) determines that the snoring is due to the nasal congestion, the supply unit (20) supplies the sleeper with air of which at least one of temperature or humidity is controlled.

According to the third aspect, the snoring due to the nasal congestion can be reduced.

A fourth aspect of the present disclosure is an embodiment of the third aspect. In the fourth aspect, when the determination unit (10) determines that the snoring is due to the nasal congestion, the supply unit (20) supplies the sleeper with humidity-controlled air.

According to the fourth aspect, the snoring due to the nasal congestion can be reduced.

A fifth aspect of the present disclosure is an embodiment of any one of the second to fourth aspects. In the fifth aspect, when the determination unit (10) determines that the snoring is due to the nasal congestion, the supply unit (20) supplies the sleeper with purified air.

According to the fifth aspect, the snoring due to the nasal congestion can be reduced.

A sixth aspect of the present disclosure is an embodiment of any one of the second to fifth aspects. In the sixth aspect, when the determination unit (10) determines that the snoring is due to the nasal congestion, the supply unit (20) supplies the sleeper with a first functional component having at least one of a vasoconstriction effect, an anti-inflammatory effect, or an immunostimulatory effect.

According to the sixth aspect, the snoring due to the nasal congestion can be reduced.

A seventh aspect of the present disclosure is an embodiment of any one of the second to sixth aspects. In the seventh aspect, when the determination unit (10) determines that the snoring is due to the constriction of the throat, the supply unit (20) supplies the sleeper with airflow that makes the sleeper take a lateral recumbent position.

According to the seventh aspect, the snoring due to the constriction of the throat can be reduced.

An eighth aspect of the present disclosure is an embodiment of any one of the first to seventh aspects. In the eighth aspect, the determination unit (10) includes a sound detector (111) configured to detect sounds emitted from the sleeper, and determines the type of the symptom based on the sounds detected by the sound detector (111).

According to the eighth aspect, it is possible to determine the type of the symptom disturbing the sleep of the sleeper based on the sounds emitted from the sleeper.

A ninth aspect of the present disclosure is an embodiment of the eighth aspect, the determination unit (10) further includes a posture detector (112) configured to detect a posture of the sleeper, and determines the type of the symptom based on the sounds detected by the sound detector (111) and the posture detected by the posture detector (112).

According to the ninth aspect, it is possible to determine the type of the symptom disturbing the sleep of the sleeper based on the sounds emitted from the sleeper and the posture of the sleeper.

A tenth aspect of the present disclosure is an embodiment of the ninth aspect. In the tenth aspect, the determination unit (10) determines that the sleeper is snoring due to the constriction of the throat when a first condition is met in which the snoring of the sleeper is reduced when the posture detected by the posture detector (112) has been changed from a supine position to a lateral recumbent position, and determines that the sleeper is snoring due to the nasal congestion when the first condition is not met.

According to the tenth aspect, it is possible to determine whether the snoring is due to nasal congestion or due to constriction of the throat.

An eleventh aspect of the present disclosure is an embodiment of any one of the first to tenth aspects. In the eleventh aspect, the symptom includes at least bruxism, and the determination unit (10) determines that the sleeper is bruxing.

According to the eleventh aspect, it is possible to select a stimulus suitable for the bruxism.

A twelfth aspect of the present disclosure is an embodiment of the eleventh aspect. In the twelfth aspect, when the determination unit (10) determines that the sleeper is bruxing, the supply unit (20) supplies the sleeper with a stimulus that reduces the bruxism of the sleeper.

According to the twelfth aspect, when the determination unit (10) determines the occurrence of the bruxism of the sleeper, the supply unit (20) supplies the sleeper with a stimulus that reduces the bruxism. Since the bruxism can be reduced, the sleep quality can be improved.

A thirteenth aspect of the present disclosure is an embodiment of the twelfth aspect. In the thirteenth aspect, when the determination unit (10) determines that the sleeper is bruxing, the supply unit (20) supplies the sleeper with a second functional component having a relaxing effect.

According to the thirteenth aspect, it is possible to reduce the bruxism.

A fourteenth aspect of the present disclosure is an embodiment of the twelfth or thirteenth aspects. In the fourteenth aspect, when the determination unit (10) determines that the sleeper is bruxing, the supply unit (20) supplies the sleeper with airflow with 1/f fluctuations.

According to the fourteenth aspect, it is possible to reduce the bruxism.

A fifteenth aspect of the present disclosure is an embodiment of any one of the first to fourteenth aspects. In the fifteenth aspect, the supply unit (20) is configured to supply the sleeper with a stimulus by two or more types of supply method, the environment control device further includes a storage (30) configured to store, as learning data, a type of the supply method through which the stimulus has been supplied to the sleeper and an effect on a reduction of the symptom, and the supply unit (20) is configured to select the supply method based on the learning data stored in the storage (30).

According to the fifteenth aspect, it is possible to reduce the symptom that disturbs sleep (e.g., snoring and bruxism) in consideration of differences of individual sleepers.

A sixteenth aspect of the present disclosure is an embodiment of any one of the first to fifteenth aspects. In the sixteenth aspect, the supply unit (20) selects a stimulus that is most effective for the symptom from among a plurality of stimuli prepared beforehand and supplies the sleeper with the selected stimulus, and if the effect on the reduction of the symptom is not recognized within predetermined time after the supply of the stimulus, the supply unit (20) selects a next most effective stimulus for the symptom from among the plurality of stimuli and supplies the sleeper with the selected stimulus.

According to the sixteenth aspect, it is possible to supply the sleeper, while checking the effects on the reduction of the symptom that disturbs sleep, with the most effective stimulus for that symptom. This can improve the reliability of the symptom reduction by the stimulus.

### BRIEF DESCRIPTION OF THE DRAWINGS

FIG. 1 is a diagram illustrating a schematic configuration of an environment control device according to an embodiment.
FIG. 2 is a flowchart showing a method of reducing a symptom that disturbs sleep by using the environment control device illustrated in FIG. 1.
FIG. 3 is a diagram illustrating an example of how the environment control device illustrated in FIG. 1 learns an effect on reduction of the symptom that disturbs sleep.

### DESCRIPTION OF EMBODIMENTS

An embodiment of the present disclosure will be described below with reference to the drawings. The following embodiment is merely a preferred example in nature, and is not intended to limit the scope, applications, or use of the invention.

### «Embodiment»

### <Configuration of Environment Control Device>

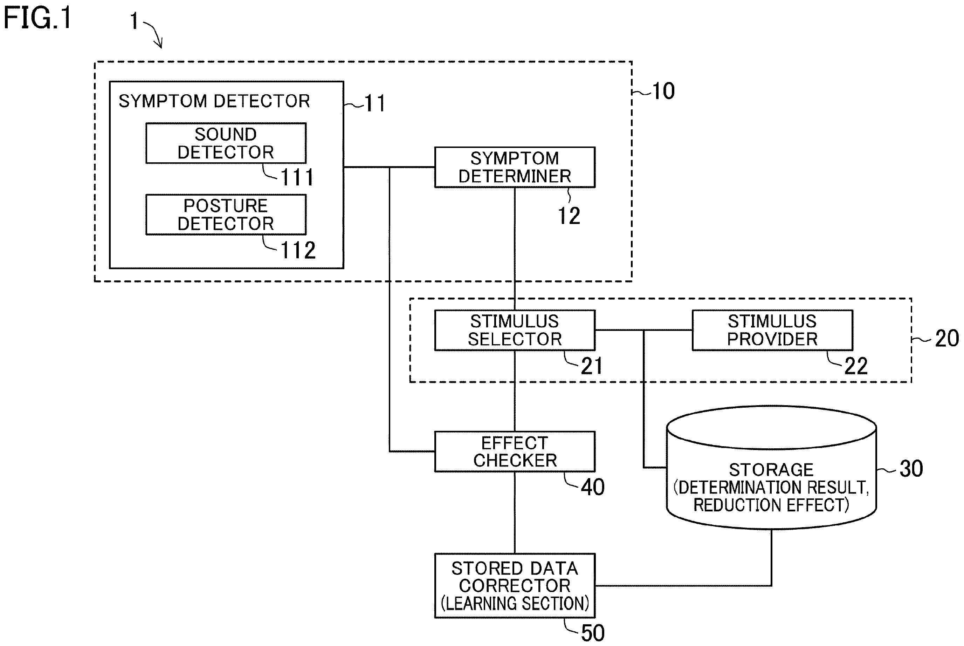

FIG. 1 is a diagram illustrating a schematic configuration of an environment control device according to this embodiment.

As illustrated in FIG. 1, the environment control device (1) has a determination unit (10) and a supply unit (20) as main components. The determination unit (10) determines the type of a symptom that disturbs sleep of a sleeper. The supply unit (20) supplies the sleeper with a stimulus that reduces the symptom, according to the type of the symptom determined by the determination unit (10).

Symptoms that disturb sleep include at least snoring. The determination unit (10) determines, for example, whether the snoring is due to nasal congestion or due to constriction of the throat. The nasal congestion determined herein is assumed to be caused by swelling of the nasal mucosa due to e.g., bacteria, viruses, and allergenic substances. Countermeasures for easing the nasal congestion include a method of removing or avoiding a causative substance of the nasal congestion and a method of reducing swelling of the nasal mucosa. Thus, the supply unit (20) of the present embodiment may supply a sleeper with air of which at least one of temperature or humidity is controlled (e.g., humidity-controlled air) when the determination unit (10) determines that the snoring is due to nasal congestion. Alternatively, the supply unit (20) may supply a sleeper with purified air when the determination unit (10) determines that the snoring is due to nasal congestion. Alternatively, the supply unit (20) may supply a sleeper with a first functional component having at least one of a vasoconstriction effect, an anti-inflammatory effect, or an immunostimulatory effect when the determination unit (10) determines that the snoring is due to nasal congestion. Alternatively, the supply unit (20) may supply a sleeper with airflow that makes the sleeper take a lateral recumbent position when the determination unit (10) determines the snoring is due to constriction of the throat.

The determination unit (10) may be configured to further determine that the sleeper is bruxing. In this case, the supply unit (20) may be configured to supply the sleeper with a stimulus that reduces the bruxism when the determination unit (10) determines that the sleeper is bruxing. Specifically, the supply unit (20) may supply the sleeper with, for example, a second functional component having a relaxing effect and/or airflow with 1/f fluctuations when the determination unit (10) determines that the sleeper is bruxing.

In this embodiment, the determination unit (10) may include a symptom detector (11) and a symptom determiner (12). The symptom detector (11) may have, for example, a sound detector (111) configured to detect sounds emitted from a sleeper, and a posture detector (112) configured to detect a posture of the sleeper. The sound detector (111) may be, for example, a smartphone, a remote control of an air conditioner, or any other similar devices, or a microphone provided in a pillow or any other products used by the sleeper. The posture detector (112) may be, for example, a posture measurement sensor or an image sensor capable of image processing. The symptom determiner (12) determines the type of the symptom that disturbs sleep of the sleeper (e.g., the type of the snoring of the sleeper and/or the occurrence of bruxism), based on the sounds detected by the sound detector (111) and/or the posture detected by the posture detector (112).

For example, the determination unit (10) may be configured to determine that the sleeper is snoring due to the constriction of the throat when a first condition is met in which the snoring of the sleeper is reduced when the posture detected by the posture detector (112) has been changed from a supine position to a lateral recumbent position, and determine that the sleeper is snoring due to the nasal congestion when the first condition is not met.

In this embodiment, the supply unit (20) may be configured to supply the sleeper with a stimulus by two or more types of supply method. The environment control device (1) may further include a storage (30) configured to store, as learning data, the type of the supply method (a determination result) through which the stimulus has been supplied to the sleeper and the effects on the reduction of the symptom that disturbs the sleep. In this case, the supply unit (20) may include a stimulus selector (21) configured to select a stimulus supply method based on the learning data stored in the storage (30), and a stimulus provider (22) configured to provide the sleeper with the stimulus selected by the stimulus selector (21). Examples of the stimulus provider (22) include an air conditioner, air purifier, a humidifier, an aroma generator, and any other similar devices.

If the environment control device (1) includes the storage (30), the environment control device (1) may further include an effect checker (40) and a stored data corrector (learning section) (50). The effect checker (40) is configured to check whether the stimulus supplied to the sleeper from the supply unit (20) shows an effect on the reduction of the symptom (e.g., snoring and/or bruxism) within predetermined time from the supply of the stimulus (i.e., whether the snoring and/or bruxism is reduced), based on the sounds detected by the sound detector (111) and/or the posture detected by the posture detector (112). The learning section (50) corrects the learning data stored in the storage (30) based on the effect on the reduction of the symptom checked by the effect checker (40). This configuration allows storage of the conditions of the stimulus that eases the symptoms, such as snoring and bruxism, in the storage (30). It is thus possible to supply the sleeper who starts to snore or brux again with the stimulus that can ease the symptom, i.e., snoring or bruxism, effectively based on the information stored in the storage (30). The learning section (50) may employ machine learning using a neural network, for example.

The environment control device (1) includes a computer, e.g., a microcomputer, which executes programs to execute functions of the symptom determiner (12), the stimulus selector (21), the effect checker (40), and the learning section (50). The storage (30) may be a recording medium, such as a computer-readable ROM, an optical disk, and a hard disk drive. The computer may be configured to be able to transmit and receive data to and from each of the symptom detector (11) and the stimulus provider (22) via a communication line or the like.

### <Method of Reducing Symptoms That Disturb Sleep>

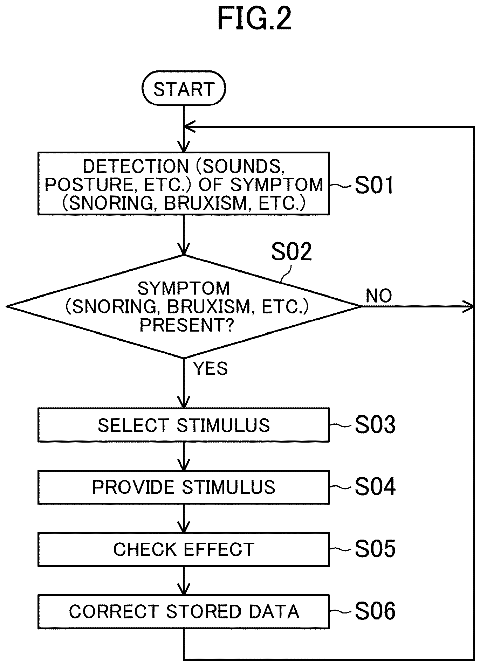

FIG. 2 is a flowchart showing a method of reducing a symptom, such as snoring and bruxism, by using the environment control device (1) illustrated in FIG. 1.

First, in Step S01, in order to detect whether the sleeper presents symptoms, such as snoring and bruxism, sounds emitted from the sleeper and/or the posture of the sleeper are detected by using the symptom detector (11) (the sound detector (111) and the posture detector (112)).

Next, in Step S02, the symptom determiner (12) determines the presence or absence of a symptom (e.g., snoring and/or bruxism) based on the sounds or posture detected in Step S01. If there is no symptom, the process returns to Step S01. If there is a symptom, the process proceeds to Step S03.

In general, snoring occurs when mucosa vibrates during breathing due to constriction of the throat or nasal congestion. In many cases, the frequency of the snoring sounds is lower than or equal to about 200 Hz or around 500 Hz. The snoring due to the constriction of the throat tends to be reduced in the lateral recumbent position, as compared to the supine position, whereas the snoring due to the nasal congestion does not have such a tendency. Thus, if the sleeper does not change their position spontaneously in Step S01, the type of the snoring may be determined by, for example, supplying cold air to the face of the sleeper to cause the sleeper to change their position from the supine position to the lateral recumbent position.

In bruxism, sounds are generated when teeth are ground. In many cases, bruxism occurs consecutively at intervals from 12.5 msec to 20.5 msec and has its peaks present in a frequency range of 2000 Hz or lower and a frequency range from 3000 Hz to 6300 Hz. Since the snoring sounds and the teeth grinding sounds have different frequency bands, the snoring sounds and the teeth grinding sounds can be discriminated from each other even when the snoring and bruxism occur at the same time.

Next, in Step S03, the stimulus selector (21) selects a stimulus to be supplied to the sleeper based on the type of the symptom (e.g., snoring and/or bruxism) determined in Step S02 and the learning data stored in the storage (30). Then, in Step S04, the stimulus provider (22) provides the sleeper with the stimulus selected in Step S03.

If the symptom determined in Step S02 is "snoring due to nasal congestion," the following stimuli may be selected, for example:
- air controlled to have the temperature and humidity (the temperature of about 20°C to about 24°C and the humidity of about 55% to about 60%) easy on the nasal cavity (which air can be supplied through an air conditioner, for example);
- purified air from which allergens, viruses and the like have been removed (which air can be supplied through an air purifier, for example); and
- the first functional component having at least one of a vasoconstriction effect, an anti-inflammatory effect, or an immunostimulatory effect (e.g., peppermint (menthol), eucalyptus, tea tree, etc., which component can be supplied through an aroma generator, for example).

The "purified air" can be outside air, which may be supplied to the sleeper by ventilation through an air conditioner or the like, if the outside air is cleaner than the room air. The "first functional component" may be supplied to only the sleeper along with vortex ring-shaped airflow generated using the air gun. Alternatively, the "first functional component" may be supplied to the sleeper by blowing airflow from an air conditioner against a wall material (for example, Hinoki or Japanese cypress) containing aromatic ingredients to promote diffusion of the aromatic ingredients.

If the symptom determined in Step S02 is "snoring due to constriction of the throat," "airflow that makes the sleeper take a lateral recumbent position" may be selected as a stimulus. For example, cold air may be supplied from an air conditioner to the face of the sleeper to cause the sleeper to turn the face opposite to the air conditioner, avoiding the cold air. It is possible to make the sleeper take the lateral recumbent position in this manner.

If the symptom determined in Step S02 is "bruxism," the following stimuli may be selected, for example:
- a second functional component having a relaxing effect (e.g., lavender (linalool), chamomile, sandalwood, cypress, neroli, etc., which component can be supplied through an aroma generator, for example); and
- airflow with 1/f fluctuations (which airflow can be supplied through an air conditioner, for example).

The "second functional component" may be supplied to only the sleeper along with vortex ring-shaped airflow generated using the air gun. Alternatively, the "second functional component" may be supplied to the sleeper by blowing airflow from an air conditioner against a wall material (for example, Hinoki or Japanese cypress) containing aromatic ingredients to promote diffusion of the aromatic ingredients.

Next, in Step S05, the effect checker (40) checks whether the stimulus provided to the sleeper in Step S04 shows an effect on the reduction of the symptom (e.g., snoring and/or bruxism) within predetermined time from the provision of the stimulus, based on the sounds detected by the sound detector (111) and/or the posture detected by the posture detector (112). Then, in Step S06, the learning section (50) corrects the learning data stored in the storage (30) based on the effect on the reduction of the symptom checked by the effect checker (40). Thereafter, the process returns to Step S01.

### <Learning of Effect on Reduction of Symptoms That Disturb Sleep>

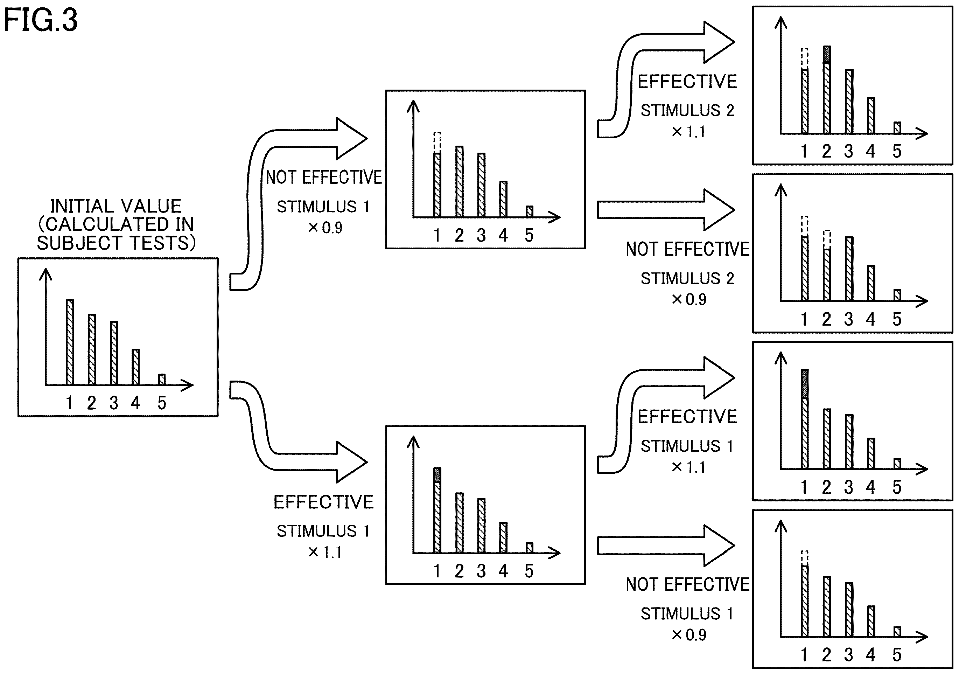

FIG. 3 is a diagram illustrating an example of how the environment control device (1) illustrated in FIG. 1 learns effects on the reduction of the symptoms, such as snoring and bruxism. In FIG. 3, the horizontal axis of each graph indicates the type of stimulus (stimuli 1 to 5 as examples), and the vertical axis indicates a symptom reduction effect value of each stimulus.

The learning data used in the environment control device (1) of this embodiment is "relationship data (hereinafter referred to as reduction effect learning data) showing reduction effects of respective stimuli for each cause of the symptom (such as snoring, bruxism, etc.)."

To generate the "reduction effect learning data," an initial value of the "reduction effect learning data" is first prepared. Specifically, subject tests are conducted in advance to see the reduction effects of the respective stimuli for each cause of the symptom. The average value of the reduction effect values obtained is stored as an initial value for each stimulus. The initial value may be prepared for each attribute (age, sex, etc.) of the subject.

FIG. 3 shows that a "stimulus 1" has the highest initial value of the "reduction effect learning data."

Subsequently, the learned value of the "reduction effect learning data" is updated while the environment control device (1) performs environment control in an actual use environment. Specifically, the symptom (such as snoring, bruxism, etc.) reduction effect is checked for each stimulus supplied to the sleeper, and the reduction effect value of each stimulus is corrected based on the result of the check. For example, the reduction effect value before the stimulus supply is multiplied by a coefficient of 0.9 if there is no reduction effect, and is multiplied by a coefficient of 1.1 if there is a reduction effect. The reduction effect value is corrected in this manner.

FIG. 3 includes a diagram showing that the reduction effect value (i.e., the initial value) of the "stimulus 1" is multiplied by the coefficient of 0.9 when the "stimulus 1" is "not effective" in the first environmental control, and a diagram showing that the reduction effect value (i.e., the initial value) of the "stimulus 1" is multiplied by the coefficient of 1.1 when the "stimulus 1" is "effective" in the first environmental control.

As a result of multiplying the reduction effect value (i.e., the initial value) of the "stimulus 1" by the coefficient of 0.9 when the "stimulus 1" is "not effective" in the first environmental control, the "stimulus 2" has the highest reduction effect value of the "reduction effect learning data." FIG. 3 includes a diagram showing that the reduction effect value of the "stimulus 2" is multiplied by the coefficient of 1.1 when the "stimulus 2" is "effective" in the second environmental control using the newly obtained "reduction effect learning data," and a diagram showing that the reduction effect value of the "stimulus 2" is multiplied by the coefficient of 0.9 when the "stimulus 2" is "not effective" in the second environmental control.

FIG. 3 also includes a diagram showing that the reduction effect value of the "stimulus 1" is multiplied by the coefficient of 1.1 when the "stimulus 1" is "effective" in the second environmental control using the "reduction effect learning data" in which the reduction effect value (i.e., the initial value) of the "stimulus 1" has been multiplied by the coefficient of 1.1, and a diagram showing that the reduction effect value of the "stimulus 1" is multiplied by the coefficient of 0.9 when the "stimulus 1" is "not effective" in the second environmental control.

These processes are repeated, which enables acquisition of the optimum "reduction effect learning data" for the sleeper. It is therefore possible to improve the reliability of the environmental control for easing the symptoms, such as snoring and bruxism.

The method of correcting the reduction effect value of each stimulus is not limited to the multiplication by a coefficient as described above. The reduction effect value may be corrected by various methods, such as the addition and/or subtraction of a predetermined value.

### -Advantages of Embodiment-

The environment control device (1) of the present embodiment described above includes: a determination unit (10) configured to determine a type of a symptom that disturbs sleep of a sleeper; and a supply unit (20) configured to supply the sleeper with a stimulus that reduces the symptom, according to the type of the symptom determined by the determination unit (10). This configuration can reduce a symptom that disturbs sleep in consideration of the cause of the symptom. The sleep quality can thus be improved.

According to the environment control device (1) of the present embodiment, the symptom includes at least snoring, and the determination unit (10) determines whether the snoring is due to nasal congestion or due to constriction of the throat, which enables the selection of stimuli suitable for the snoring due to nasal congestion and the snoring due to constriction of the throat.

According to the environment control device (1) of the present embodiment, when the determination unit (10) determines that the snoring is due to the nasal congestion, the supply unit (20) supplies the sleeper with air of which at least one of temperature or humidity is controlled (e.g., humidity-controlled air), which can reduce the snoring due to the nasal congestion.

According to the environment control device (1) of the present embodiment, when the determination unit (10) determines that the snoring is due to the nasal congestion, the supply unit (20) supplies the sleeper with purified air, which can reduce the snoring due to the nasal congestion.

According to the environment control device (1) of the present embodiment, when the determination unit (10) determines the snoring is due to the nasal congestion, the supply unit (20) supplies the sleeper with a first functional component having at least one of a vasoconstriction effect, an anti-inflammatory effect, or an immunostimulatory effect, which can reduce the snoring due to the nasal congestion.

According to the environment control device (1) of the present embodiment, when the determination unit (10) determines the snoring is due to the constriction of the throat, the supply unit (20) supplies the sleeper with airflow that makes the sleeper take a lateral recumbent position, which can reduce the snoring due to the constriction of the throat.

According to the environment control device (1) of the present embodiment, the determination unit (10) includes a sound detector (111) configured to detect sounds emitted from the sleeper, and determines the type of the symptom disturbing sleep of the sleeper based on the sounds detected by the sound detector (111), which enables determination of the type of the symptom disturbing the sleep of the sleeper based on the sounds emitted from the sleeper.

According to the environment control device (1) of the present embodiment, the determination unit (10) further includes a posture detector (112) configured to detect a posture of the sleeper, and determines the type of the symptom disturbing sleep of the sleeper based on the sounds detected by the sound detector (111) and the posture detected by the posture detector (112), which enables determination of the type of the symptom disturbing the sleep of the sleeper based on the sounds emitted from the sleeper and the posture of the sleeper.

According to the environment control device (1) of the present embodiment, the determination unit (10) may determine that the sleeper is snoring due to the constriction of the throat when a first condition is met in which the snoring of the sleeper is reduced when the posture detected by the posture detector (112) has been changed from a supine position to a lateral recumbent position, and may determine that the sleeper is snoring due to the nasal congestion when the first condition is not met. This configuration enables determination of whether the snoring is due to nasal congestion or due to constriction of the throat.

According to the environment control device (1) of the present embodiment, the symptom disturbing the sleep includes at least bruxism, and the determination unit (10) determines that the sleeper is bruxing, which enables the selection of a stimulus suitable for the bruxism. In this case, when the determination unit (10) determines that the sleeper is bruxing, the supply unit (20) supplies the sleeper with a stimulus that reduces the bruxism, which makes it possible to reduce the bruxism and therefore improve the sleep quality.

According to the environment control device (1) of the present embodiment, when the determination unit (10) determines that the sleeper is bruxing, the supply unit (20) supplies the sleeper with a second functional component having a relaxing effect, which can reduce the bruxism.

According to the environment control device (1) of the present embodiment, when the determination unit (10) determines that the sleeper is bruxing, the supply unit (20) supplies the sleeper with airflow with 1/f fluctuations, which can reduce the bruxism.

According to the environment control device (1) of the present embodiment, the supply unit (20) may be configured to supply the sleeper with a stimulus by two or more types of supply method. In this case, the environment control device (1) may further include a storage (30) configured to store, as learning data, the type of the supply method through which the stimulus has been supplied to the sleeper and the effects on the reduction of the symptom disturbing the sleep, and the supply unit (20) may be configured to select a supply method based on the learning data stored in the storage (30). This configuration can reduce the symptom that disturbs sleep (e.g., snoring and bruxism) in consideration of differences of individual sleepers.

As described above, the environment control device (1) of the present embodiment detects symptoms, such as snoring and bruxism during sleep, and further analyzes and determines the cause of the symptoms (e.g., snoring due to nasal congestion and snoring due to constriction of the throat) based on sound information or other information in the environment. This configuration enables selection of a stimulus that is most effective for the cause of the symptom from among a plurality of stimuli prepared beforehand, and supply of the selected stimulus to the sleeper for predetermined time (e.g., about several minutes). Moreover, in the control by the environment control device (1), whether there is a reduction effect (whether the symptoms, such as snoring and bruxism, have been reduced or not) within predetermined time after the stimulus supply is checked: if the reduction effect is not recognized, the next most effective stimulus is selected sequentially. It is therefore possible to achieve the environmental control less influenced by the individual differences.

### <<Other Embodiments>>

In the above embodiment, the environment control device (1) determines both of the type of snoring and the occurrence of the bruxism as the types of the symptoms that disturb sleep of the sleeper. Instead, the environment control device (1) may determine either one of the type of the snoring or the occurrence of the bruxism. Alternatively, the occurrence of other symptoms, such as sleep apnea syndrome, may be determined in addition to the type of snoring and/or the occurrence of the bruxism.

The symptom detector (11) includes both of the sound detector (111) and the posture detector (112), but may include only the sound detector (111). In this case, the learning section (50) performs machine learning using only sounds detected by the sound detector (111) to estimate the symptoms, such as snoring and bruxism. Alternatively, the symptom detector (11) may include a means for detecting another index, such as the body temperature of the sleeper, in addition to the sound detector (111) and the posture detector (112).

While the embodiments (including other embodiments) have been described above, it will be understood that various changes in form and details can be made without departing from the spirit and scope of the claims. The embodiments (including other embodiments) may be combined and replaced with each other without deteriorating intended functions of the present disclosure. In addition, the expressions of "first," "second," ... described above are used to distinguish the terms to which these expressions are given, and do not limit the number and order of the terms.

### INDUSTRIAL APPLICABILITY

As can be seen from the foregoing description, the present disclosure is useful for an environment control device.

### DESCRIPTION OF REFERENCE CHARACTERS

- 1: Environment Control Device
- 10: Determination Unit
- 11: Symptom Detector
- 111: Sound Detector
- 112: Posture Detector
- 12: Symptom Determiner
- 20: Supply Unit
- 21: Stimulus Selector
- 22: Stimulus Provider
- 30: Storage
- 40: Effect Checker
- 50: Learning Section

## Claims

1. An environment control device, comprising:
a determination unit (10) configured to determine a type of a symptom that disturbs sleep of a sleeper; and
a supply unit (20) configured to supply the sleeper with a stimulus that reduces the symptom, according to the type of the symptom determined by the determination unit (10).

2. The environment control device of claim 1, wherein
the symptom includes at least snoring, and
the determination unit (10) determines whether the snoring is due to nasal congestion or due to constriction of the throat.

3. The environment control device of claim 2, wherein
when the determination unit (10) determines that the snoring is due to the nasal congestion, the supply unit (20) supplies the sleeper with air of which at least one of temperature or humidity is controlled.

4. The environment control device of claim 3, wherein
when the determination unit (10) determines that the snoring is due to the nasal congestion, the supply unit (20) supplies the sleeper with humidity-controlled air.

5. The environment control device of any one of claims 2 to 4, wherein when the determination unit (10) determines that the snoring is due to the nasal congestion, the supply unit (20) supplies the sleeper with purified air.

6. The environment control device of any one of claims 2 to 5, wherein when the determination unit (10) determines that the snoring is due to the nasal congestion, the supply unit (20) supplies the sleeper with a first functional component having at least one of a vasoconstriction effect, an anti-inflammatory effect, or an immunostimulatory effect.

7. The environment control device of any one of claims 2 to 6, wherein when the determination unit (10) determines that the snoring is due to the constriction of the throat, the supply unit (20) supplies the sleeper with airflow that makes the sleeper take a lateral recumbent position.

8. The environment control device of any one of claims 1 to 7, wherein
the determination unit (10)
includes a sound detector (111) configured to detect sounds emitted from the sleeper, and
determines the type of the symptom based on the sounds detected by the sound detector (111).

9. The environment control device of claim 8, wherein
the determination unit (10)
further includes a posture detector (112) configured to detect a posture of the sleeper, and
determines the type of the symptom based on the sounds detected by the sound detector (111) and the posture detected by the posture detector (112).

10. The environment control device of claim 9, wherein
the determination unit (10)
determines that the sleeper is snoring due to the constriction of the throat when a first condition is met in which the snoring of the sleeper is reduced when the posture detected by the posture detector (112) has been changed from a supine position to a lateral recumbent position, and
determines that the sleeper is snoring due to the nasal congestion when the first condition is not met.

11. The environment control device of any one of claims 1 to 10, wherein
the symptom includes at least bruxism, and
the determination unit (10) determines that the sleeper is bruxing.

12. The environment control device of claim 11, wherein
when the determination unit (10) determines that the sleeper is bruxing, the supply unit (20) supplies the sleeper with a stimulus that reduces the bruxism of the sleeper.

13. The environment control device of claim 12, wherein
when the determination unit (10) determines that the sleeper is bruxing, the supply unit (20) supplies the sleeper with a second functional component having a relaxing effect.

14. The environment control device of claim 12 or 13, wherein
when the determination unit (10) determines that the sleeper is bruxing, the supply unit (20) supplies the sleeper with airflow with 1/f fluctuations.

15. The environment control device of any one of claims 1 to 14, wherein
the supply unit (20) is configured to supply the sleeper with a stimulus by two or more types of supply method,
the environment control device further includes a storage (30) configured to store, as learning data, a type of the supply method through which the stimulus has been supplied to the sleeper and an effect on a reduction of the symptom, and
the supply unit (20) is configured to select the supply method based on the learning data stored in the storage (30).

16. The environment control device of any one of claims 1 to 15, wherein
the supply unit (20) selects a stimulus that is most effective for the symptom from among a plurality of stimuli prepared beforehand and supplies the sleeper with the selected stimulus, and if the effect on the reduction of the symptom is not recognized within predetermined time after the supply of the stimulus, the supply unit (20) selects a next most effective stimulus for the symptom from among the plurality of stimuli and supplies the sleeper with the selected stimulus.
